(19) 

Europäisches
Patentamt
European
Patent Office
Office européen
des brevets

(11) **EP 3 764 096 A1**

(12) **EUROPEAN PATENT APPLICATION**
published in accordance with Art. 153(4) EPC

(43) Date of publication:
**13.01.2021 Bulletin 2021/02**

(21) Application number: **19764779.5**

(22) Date of filing: **01.03.2019**

(51) Int Cl.:
*G01N 33/483* (2006.01)     *G01N 21/64* (2006.01)
*G01N 33/53* (2006.01)     *G06T 1/00* (2006.01)

(86) International application number:
**PCT/JP2019/007984**

(87) International publication number:
**WO 2019/172097 (12.09.2019 Gazette 2019/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.03.2018 JP 2018040309**

(71) Applicant: **KONICA MINOLTA, INC.**
**Tokyo**
**100-7015 (JP)**

(72) Inventor: **OZAKI, Yuichi**
**Tokyo 100-7015 (JP)**

(74) Representative: **Henkel & Partner mbB**
**Patentanwaltskanzlei, Rechtsanwaltskanzlei**
**Maximiliansplatz 21**
**80333 München (DE)**

(54) **IMAGE PROCESSING METHOD, IMAGE PROCESSING DEVICE, AND PROGRAM**

(57) The present invention addresses the problem of providing an image processing method, an image processing device, and a program capable of quantitatively evaluating the expression level of a specific protein more accurately in a tissue specimen in which fluorescent staining of the specific protein by means of a fluorescent substance, and dye staining for visualizing a cell or a region of interest in a cell, have been carried out in a multiple manner. The image processing method includes: an input step of inputting a fluorescence image and a cell morphology image obtained by capturing images of a tissue specimen in which specific biological material has been stained using a fluorescent substance capable of binding to the specific biological material, and in which a cell has been stained using a dye capable of being observed under visible light; a brightness value calculating step of extracting from the fluorescence image a specific region containing a bright spot, and calculating a brightness value of the specific region; and a brightness value adjusting step of adjusting the brightness value of the specific region on the basis of dye information in the cell morphology image.

FIG.5

EP 3 764 096 A1

**Description**

TECHNOLOGICAL FIELD

The present invention relates to an image processing method, an image processing device, and a program, especially to image processing applied to pathological diagnosis.

BACKGROUND ART

In pathological diagnosis, quantification of an expression level of a biological material overexpressing in a tissue section provides very important information for predicting a prognosis and for deciding a treatment plan afterward. In particular, quantitative assessment of an expression level of oncoprotein in cells provides a vital clue in determining malignancy of cancer.

Traditionally, a technique of assessing an expression level of protein based on the number of bright spots have been used to quantitatively assess an expression level of specific protein. A tissue specimen is stained with fluorescent substance-assembled particles targeted on the specific protein. Fluorescent bright spots are extracted from a fluorescence image which is obtained by imaging the tissue specimen. Then the number of bright spots is measured. However, even if it appears to be a single bright spot on the fluorescence image, in fact, a plurality of fluorescent substance-assembled particles sometimes cluster together. It is difficult to accurately assess an expression level just by measuring the number of bright spots.

In view of this, for example, Patent document 1 discloses a technique of assessing an expression level of a specific protein based on the number of fluorescent substance-assembled particles. Specifically, fluorescent bright spots are first extracted from a fluorescence image. Brightness distribution of the extracted fluorescent bright spots is analyzed. Thereby an average brightness value per particle is calculated. The number of particles contained in each bright spot is calculated based on the calculated average brightness value and a brightness value of each bright spot. An expression level of specific protein is assessed by comparing the numbers of particles. This technique brings more reliable result as compared with a case in which just the number of bright spots is measured.

PATENT DOCUMENT

Patent Document 1: WO 2012/029342 A

SUMMARY OF THE INVENTION

PROBLEMS TO BE SOLVED BY THE INVENTION

In conjunction with fluorescent staining of specific protein, a tissue specimen is commonly stained with a dye using a staining reagent capable of being observed under a visible light to identify a cell or a region of interest in a cell. The dye used in staining sometimes absorbs excitation light from a light source or fluorescence from a fluorescent substance. It reduces a brightness value to be detected. Decreased accuracy in detecting the brightness value decreases accuracy in detecting the expression level of specific protein. It is a problem.

The present invention was made in view of the above problem. It is an object of the present invention to provide an image processing method, an image processing device, and a program that quantitatively evaluates an expression level of specific protein in a tissue specimen more accurately, the tissue specimen having been stained through fluorescent staining of specific protein with a fluorescent substance and through dye staining for visualizing a cell or a region of interest in a cell.

MEANS FOR SOLVING PROBLEMS

To solve the above problem, the image processing method according to claim 1 includes:

inputting a fluorescence image and a morphological cell image that are obtained by imaging a tissue specimen in which:

a specific biological material is stained with a fluorescent substance capable of binding with the specific biological material; and
cells are stained with a dye capable of being observed under visible light;

extracting a predetermined region including a bright spot from the fluorescence image and calculating a brightness value of the predetermined region; and adjusting the brightness value of the predetermined region based on dye information of the morphological cell image.

The invention according to claim 2 is the image processing method according to claim 1, further including:

calculating a dye amount of a dye used to stain the morphological cell image for each pixel in the morphological cell image,
wherein, in adjusting the brightness value, the dye amount is used as the dye information.

The invention according to claim 3 is the image processing method according to claim 2, wherein in calculating the dye amount, the dye amount is estimated in a color deconvolution method based on a dye base of each pixel in the morphological cell image, and a dye density image in which the dye amount is distributed in relation to pixels of the morphological cell image is generated, and

in adjusting the brightness value, the brightness value is adjusted based on the dye density image.

The invention according to claim 4 is the image processing method according to claim 1, further including:

calculating chroma of a dye used to stain the morphological cell image for each pixel in the morphological cell image,

wherein, in adjusting the brightness value, the brightness value is adjusted using the chroma as the dye information.

The invention according to claim 5 is the image processing method according to claim 1, wherein in adjusting the brightness value, RGB values are used as the dye information.

The invention according to claim 6 is the image processing method according to any one of claims 1 to 5, wherein

the tissue specimen is stained with a fluorescent substance and a dye,

the fluorescent substance consists of a single kind of fluorescent substance or multiple kinds of fluorescent substances having different wavelength characteristics, the dye consists of a single kind of dye or multiple kinds of dyes having different wavelength characteristics, and

in adjusting the brightness value, the brightness value is adjusted for each combination of the fluorescent substance and the dye based on the dye information of the dye.

The invention according to claim 7 is the image processing method according to any one of claims 1 to 6, wherein

the tissue specimen is stained with multiple kinds of dyes having different wavelength characteristics, and

in adjusting the brightness value, the brightness value is adjusted differently depending on staining order of the multiple kinds of dyes.

An image processing method according to claim 8 includes:

inputting a fluorescence image and a morphological cell image that are obtained by imaging a tissue specimen in which:

a specific biological material is stained with a fluorescent substance capable of binding with the specific biological material; and
cells are stained with a dye capable of being observed under visible light;

extracting a predetermined region including a bright spot from the fluorescence image and calculating a brightness value of the predetermined region;
calculating a feature amount that indicates an expression level of the specific biological material based on the brightness value of the predetermined region; and
adjusting the feature amount based on dye information of the morphological cell image.

The invention according to claim 9 is the image processing method according to any one of claims 1 to 8, wherein

the fluorescent substance consists of fluorescent substance-assembled particles in which a plurality of fluorescent substances are assembled.

An image processing device according to claim 10 includes:

an input unit that inputs a fluorescence image and a morphological cell image that are obtained by imaging a tissue specimen in which:

a specific biological material is stained with a fluorescent substance capable of binding with the specific biological material; and
cells are stained with a dye capable of being observed under visible light;

a brightness calculator that extracts a predetermined region including a bright spot from the fluorescence image and calculates a brightness value of the predetermined region; and
a brightness adjuster that adjusts the brightness value of the predetermined region based on dye information of the morphological cell image.

A program according to claim 11 makes a computer in an image processing device function as:

an input unit that inputs a fluorescence image and a morphological cell image that are obtained by imaging a tissue specimen in which:

a specific biological material is stained with a fluorescent substance capable of binding with the specific biological material; and
cells are stained with a dye capable of being observed under visible light;

a brightness calculator that extracts a predetermined region including a bright spot from the fluorescence image and calculates a brightness value of the predetermined region; and
a brightness adjuster that adjusts the brightness value of the predetermined region based on dye information of the morphological cell image.

ADVANTAGEOUS EFFECTS OF INVENTION

The present invention provides an image processing method, an image processing device, and a program that quantitatively evaluates an expression level of specific protein in a tissue specimen more accurately, the tissue specimen having been stained through fluorescent staining of specific protein with a fluorescent substance and through dye staining for visualizing a cell or a region of interest in a cell.

BRIEF DESCRIPTION OF THE DRAWINGS

FIG. 1 shows system configuration of a pathological diagnosis support system to which a biological material quantification method of the present invention is applied.
FIG. 2 is a block diagram showing functional configuration of an image processing device in FIG. 1.
FIG. 3 shows an example of a bright field image.
FIG. 4 shows an example of a fluorescence image.
FIG. 5 is a flow chart showing image analysis processing 1 in the first embodiment.
FIG. 6 is a flow chart showing details of processing of Step S2 in FIG. 5.
FIG. 7A shows an image in which a bright field image is extracted.
FIG. 7B shows an image in which cell nucleuses are extracted.
FIG. 8 is a flow chart showing details of processing of Step S4 in FIG. 5.
FIG. 9 is a flow chart showing details of processing of Step S8 in FIG. 5.
FIG. 10A shows an image in which a fluorescence image is extracted.
FIG. 10B shows an image in which bright spot regions are extracted.
FIG. 11 is an example of a brightness distribution curve.
FIG. 12 is a flow chart showing image analysis processing 2 in Modification 1.
FIG. 13 is a flow chart showing image analysis processing 3 in Modification 2.
FIG. 14 is a flow chart showing image analysis processing 4 in the second embodiment.

EMBODIMENTS FOR CARRYING OUT THE INVENTION

FIRST EMBODIMENT

Hereinafter, the first embodiment for carrying out the present invention will be described with reference to the drawings, but the present invention is not limited thereto.

CONFIGURATION OF PATHOLOGICAL DIAGNOSIS SUPPORT SYSTEM 100

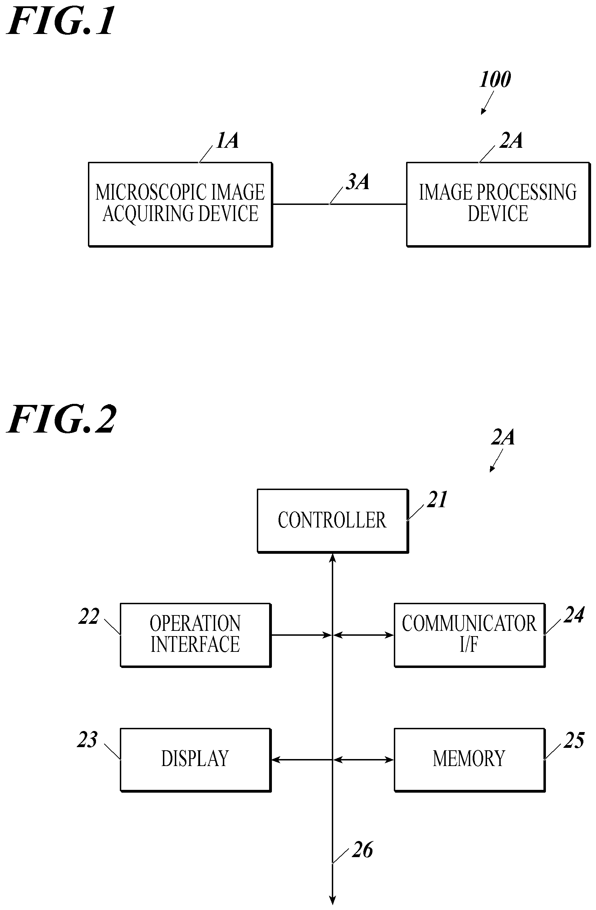

FIG. 1 shows an overall configuration example of a pathological diagnosis support system 100 to which an image processing method of the invention is applied.

The pathological diagnosis support system 100 acquires a microscopic image of a tissue specimen stained with predetermined staining reagents and analyzes the acquired microscopic image. The system quantitatively evaluates an amount of a specific biological material appearing in the tissue specimen of an observation target.
As shown in FIG. 1, the pathological diagnosis support system 100 is configured such that the microscopic image acquiring device 1A and the image processing device 2A are connected so as to be able to transmit and receive data via an interface, such as a cable 3A. The connection between the microscope image acquiring device 1A and the image processing device 2A is not particularly limited. For example, the microscope image acquiring device 1A and the image processing device 2A may be connected via a LAN (Local Area Network) or may be connected wirelessly.
The microscopic image acquiring device 1A is a well-known optical microscope with a camera which obtains the microscopic image of the tissue specimen on a slide placed on a slide fixing stage and sends it to the image processing device 2A.
The microscopic image acquiring device 1A includes an irradiating unit, an image forming unit, an imaging unit, a communicator I/F, and the like. The irradiating unit includes a light source, a filter, and the like, and irradiates the tissue specimen on the slide placed on the slide fixing stage with light. The image forming unit includes an ocular lens, an object lens, and the like, and forms an image of transmitted light, reflected light, or fluorescence from the tissue specimen on the slide due to the irradiated light. The imaging unit is a camera provided in a microscope which includes a CCD (Charge Coupled Device) sensor, and the like, and captures an image on an image forming face formed by the image forming unit to generate digital image data of the microscopic image. The communicator I/F transmits the image data of the generated microscopic image to the image processing device 2A. In this embodiment, the microscopic image acquiring device 1A includes a bright field unit which is combination of the irradiating unit and the image forming unit suitable for bright field observation and a fluorescent unit which is combination of the irradiating unit and the image forming unit suitable for fluorescence observation. The bright field and fluorescence are switched by switching the units.
The microscopic image acquiring device 1A is not limited to a microscope having a camera. For example, a virtual microscope slide creating device which scans a slide on a slide fixing stage of a microscope and obtains a microscopic image of the entire tissue specimen may be used (for example, see Japanese Patent Application Laid-Open Publication No. 2002-514319). According to the virtual microscope slide creating device, image data can be obtained with which the entire image of the tissue specimen on the slide can be viewed at once on a display.
The image processing device 2A analyzes the microscopic image transmitted from the microscopic image acquiring device 1A to calculate amounts of specific biological materials appearing in the tissue specimen of the observation target.

FIG. 2 shows an example of a functional configuration of the image processing device 2A. As shown in FIG. 2, the image processing device 2A includes a controller 21, an operation interface 22, a display 23, a communicator I/F 24, a memory 25, and the like. These components are connected through a bus 26.

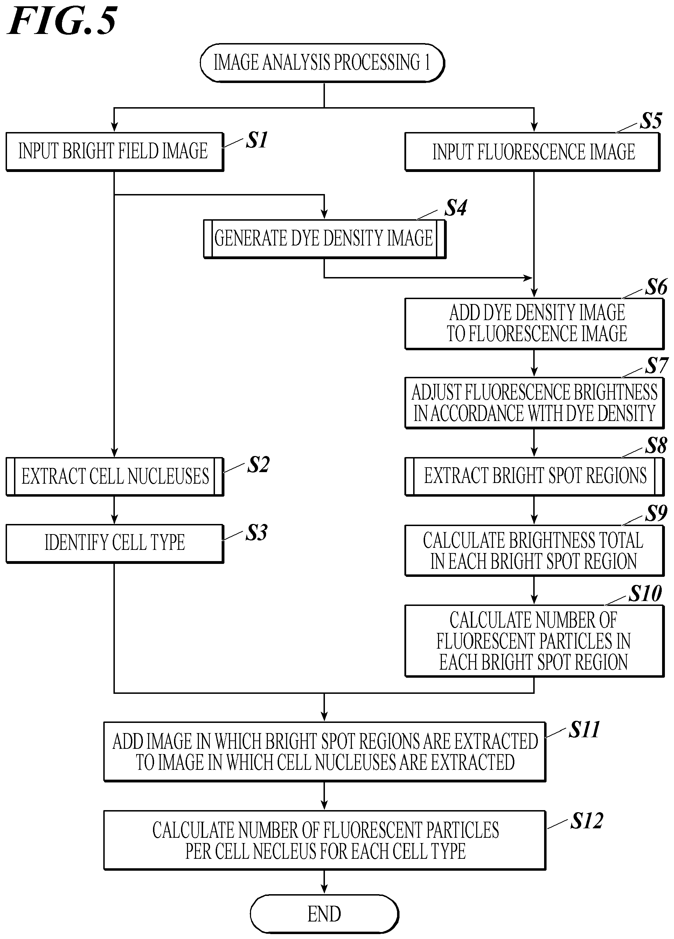

The controller 21 includes a CPU (Central Processing Unit), a RAM (Random Access Memory), and the like, performs various processing in coordination with various programs stored in the memory 25, and collectively controls operation of the image processing device 2A. For example, the controller 21 performs image analysis process 1 (see FIG. 5) in coordination with programs stored in the memory 25, and functions as means for executing input, brightness calculation, brightness adjustment, dye amount calculation, and chroma calculation.

The operation interface 22 includes a keyboard provided with character input keys, numeric input keys, and various function keys and a pointing device such as a mouse, and outputs depression signals of the pressed keys of the keyboard and operation signals of the mouse as the input signal to the controller 21.

The display 23 includes, for example, a monitor such as a CRT (Cathode Ray Tube), an LCD (Liquid Crystal Display), and the like, and displays various screens according to an instruction of a display signal input from the controller 21. In this embodiment, the display 23 functions as an output unit that outputs result of image analysis.

The communicator I/F 24 is an interface for transmitting and receiving data to and from external devices such as the microscopic image acquiring device 1A. The communicator I/F 24 together with the controller 21 function as a means for performing input of a bright field image and a fluorescence image.

The memory 25 includes, for example, an HDD (Hard Disk Drive), a nonvolatile semiconductor memory, and the like. The memory 25 stores various programs and various pieces of data as described above.

Other than the above, the image processing device 2A may include a LAN adaptor, a router, and the like, and may be connected to external devices through a communication network such as a LAN.

Preferably, the image processing device 2A in the embodiment performs analysis using a bright field image and a fluorescence image transmitted from the microscopic image acquiring device 1A.

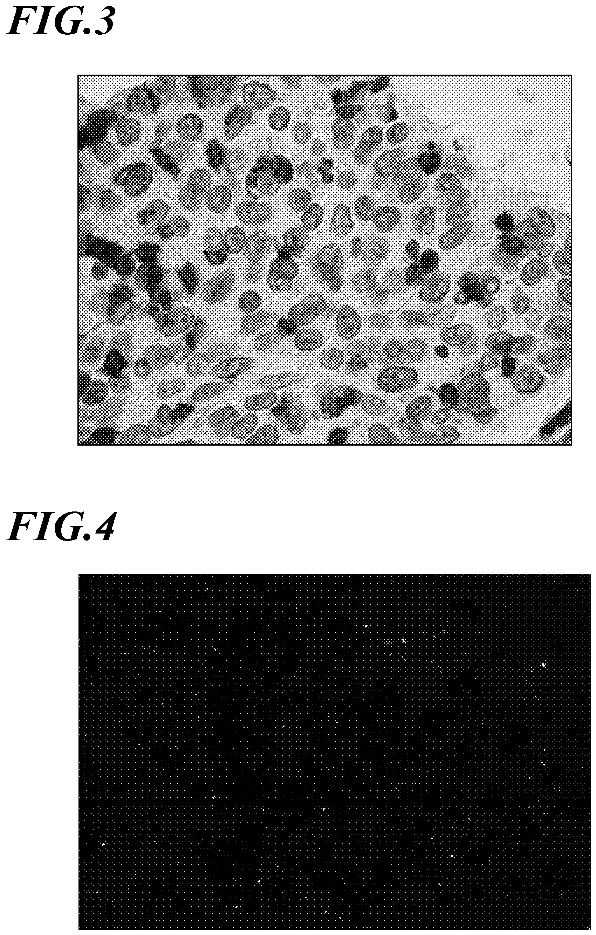

The bright field image is a microscopic image acquired by magnifying and imaging a tissue specimen in a bright field in the microscopic image acquiring device 1A after the tissue specimen is stained through H (hematoxylin) staining and DAB staining. The bright field image represents morphology of cells in the tissue specimen. Hematoxylin is a bluish violet dye and stains cell nuclei, bony tissue, a portion of cartilaginous tissue, serous components, and the like (basophilic tissue and the like). DAB staining is an enzyme antibody method. As a labeling enzyme, peroxidase is used. As a chromogenic substrate, diaminobenzidine (DAB) which is colored brown by peroxidase is used. FIG. 3 shows an example of the bright field image obtained by imaging a tissue specimen stained through H staining and DAB staining. In FIG. 3, immune cells are stained through DAB staining.

The tissue specimen is stained with a staining reagent that includes particles (hereinafter referred to as "fluorescent substance-assembled particles") containing a fluorescent substance bound with a biological material-recognizing portion that specifically binds and/or reacts with a specific biological material. The microscopic image acquiring device 1A irradiates the stained tissue specimen with excitation light of a predetermined wavelength to make fluorescent substance-assembled particles emit light (fluorescence). The fluorescence image is a microscopic image obtained by magnifying and imaging the fluorescence. That is, the fluorescence appearing in the fluorescence image indicates expression of the specific biological material corresponding to the biological material-recognizing portion in the tissue specimen. FIG. 4 shows an example of the fluorescence image.

OBTAINING FLUORESCENCE IMAGE

A method of obtaining the fluorescence image will be explained in detail. The staining reagent (fluorescent substance-assembled particles) for obtaining the fluorescence image and a method of staining the tissue specimen with the staining reagent will also be explained.

FLUORESCENT SUBSTANCE

Examples of the fluorescent substance used in the staining reagent for obtaining the fluorescence image include a fluorescent organic dye and a quantum dot (semiconductor particles). Preferably, the substance exhibits emission of visible to near infrared rays having a wavelength within the range from 400 to 1100 nm when excited by ultraviolet to near infrared rays having a wavelength within the range from 200 to 700 nm.

Examples of the fluorescent organic dye include fluorescein dye molecules, rhodamine dye molecules, Alexa Fluor (manufactured by Invitrogen Corporation) dye molecules, BODIPY (manufactured by Invitrogen Corporation) dye molecules, cascade dye molecules, coumarin dye molecules, eosin dye molecules, NBD dye molecules, pyrene dye molecules, Texas Red dye molecules and cyanine dye molecules.

Specific examples thereof include 5-carboxy-fluorescein, 6-carboxy-fluorescein, 5,6-dicarboxy-fluorescein, 6-carboxy-2',4,4',5',7,7'-hexachlorofluorescein, 6-carboxy-2',4,7,7'-tetrachlorofluorescein, 6-carboxy-4',5'-dichloro-2',7'-dimethoxyfluorescein, naphthofluorescein, 5-carboxy-rhodamine, 6-carboxy-rhodamine, 5,6-dicarboxy-rhodamine, rhodamine 6G, tetramethylrhodamine, X-rhodamine, and Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Al'xa Fluor 568, Alexa Fluor 594, Alexa Fluor 610, Alexa Fluor 633, Alexa Fluor 635, Alexa Fluor 647, Alexa Fluor 660, Alexa Fluor 680, Alexa Fluor 700, Alexa Fluor 750, BODIPY FL, BODIPY TMR, BODIPY 493/503, BODIPY 530/550, BODIPY 558/568, BODIPY 564/570, BODIPY 576/589, BODIPY 581/591, BODIPY 630/650, BODIPY 650/665 (the above are manufactured by Invitrogen Corporation), methoxycoumalin, eosin, NBD, pyrene, Cy5, Cy5.5 and Cy7. These can be used individually, or be used by mixing several kinds thereof.

Usable examples of the quantum dot include quantum dots respectively containing, as a component, II-VI compounds, III-V compounds, and IV elements (called "II-VI quantum dot", "III-V quantum dot" and "IV quantum dot", respectively). These can be used individually, or be used by mixing several kinds thereof.

Specific examples thereof include but are not limited to CdSe, CdS, CdTe, ZnSe, ZnS, ZnTe, InP, InN, InAs, InGaP, GaP, GaAs, Si and Ge.

A quantum dot having a core of any of the above quantum dots and a shell provided thereon can also be used. Hereinafter, as a notation for the quantum dot having a shell, when the core is CdSe and the shell is ZnS, the quantum dot is noted as CdSe/ZnS. Usable examples of the quantum dot include but are not limited to CdSe/ZnS, CdS/ZnS, InP/ZnS, InGaP/ZnS, Si/SiO2, Si/ZnS, Ge/GeO2, and Ge/ZnS.

A quantum dot surface-treated with an organic polymer or the like may be used as needed. Examples thereof include CdSe/ZnS having a surface carboxy group (manufactured by Invitrogen Corporation) and CdSe/ZnS having a surface amino group (manufactured by Invitrogen Corporation).

FLUORESCENT SUBSTANCE-ASSEMBLED PARTICLES

The fluorescent substance-assembled particles in the embodiment are particles in which a fluorescent substance is dispersed in the particles or is adsorbed on the surface of the particles. The fluorescent substance and the particles may or may not be chemically bound with each other.

The material composing the particles is not particularly limited, and examples thereof include polystyrene, polyactate acid, silica, melamine, and the like.

The fluorescent substance-assembled particles in this embodiment can be produced by known methods. For example, fluorescent organic dye-containing silica particles can be synthesized by referring to the synthesis of FITC-containing silica particles described in Langmuir, vol. 8, page 2921 (1992). A variety of fluorescent organic dye-containing silica particles can be synthesized by using any desired fluorescent organic dye instead of FITC.

Quantum dot-containing silica particles can be synthesized by referring to the synthesis of CdTe-containing silica particles described in New Journal of Chemistry, vol. 33, page 561 (2009).

Fluorescent organic dye-containing polystyrene particles can be produced by using a copolymerization method using an organic dye having a polymerizable functional group described in U.S. Patent No. 4,326,008 (1982) or a method of impregnating a fluorescent organic dye into polystyrene particles described in U.S. Patent No. 5,326,692 (1992).

Quantum dot-containing polymer particles can be produced by using the method of impregnating a quantum dot into polystyrene particles described in Nature Biotechnology, vol. 19, page 631 (2001).

The average particle size of fluorescent substance-assembled particles used in the embodiment is not limited. Those with large particle size have less access to antigen. Those with small particle size have a lower brightness value so that signals of fluorescent particles are buried in background noise (camera noise or auto fluorescence of cells). Therefore, those of about 50-300nm are suitable.

The average particle diameter is obtained by capturing electronic microscope pictures using the scanning electron microscope (SEM), measuring cross sectional areas of a sufficient number of particles, and obtaining a diameter of a circle having an area of each measured value as a particle diameter. In the present application, the average particle diameter is a calculated average of particle diameters from 1000 particles. The coefficient of variation is also a value calculated from particle diameter distribution of 1000 particles.

There is a positive correlation between a brightness value and particle size of one fluorescent particle. The shapes of all the distribution curves indicate almost the same t distribution.

BINDING OF BIOLOGICAL MATERIAL-RECOGNIZING PORTION AND FLUORESCENT SUBSTANCE-ASSEMBLED PARTICLES

The biological material-recognizing portion in this embodiment is a portion which specifically binds and/or reacts with a target biological material. The target biological material is not particularly limited as long as there exists a substance that specifically binds with it. Representative examples of the substance include protein (peptide), nucleic acid (oligonucleotide, polynucleotide), an antibody, and the like. Examples of a substance that binds with the target biological material include an antibody which recognizes the protein as an antigen, another protein which specifically binds with the protein, nucleic acid including a base sequence which hybridizes with the nucleic acid, and the like. Specific examples include anti-HER2 antibody which specifically binds with the HER2 which is a protein on the surface of the cell, anti-ER antibody which specifically binds with the estrogen receptor (ER) in the cell nucleus, anti-actin antibody which specifically binds with the actin forming the cytoskeleton, and the like. Among the above, anti-HER2 antibody and anti-ER antibody bound with the fluorescent substance-assembled particles are preferable because they can be used for selecting drug administration to treat breast cancer.

The binding form between the biological material-recognizing portion and the fluorescent particles is not particularly limited, and examples include, covalent bond, ionic bond, hydrogen bond, coordinate bond, physical adsorption, chemical adsorption, and the like. Binding with strong binding force such as covalent bond is preferable in view of stability of binding.

There may be an organic molecule connecting the biological material-recognizing portion and the fluorescent particles. For example, in order to suppress nonspecific absorption with the biological material, a polyethyleneglycol chain, such as SM (PEG) 12 manufactured by Thermo Scientific, can be used.

In a case in which the biological material-recognizing portion is bound with the fluorescent substance-containing silica particles, the same process can be applied no matter whether the fluorescent substance is the fluorescent organic dye or the quantum dot. For example, a silane coupling agent which is a compound widely used for binding inorganic material and organic material can be used. The silane coupling agent is a compound including an alkoxysilyl group providing a silanol group with hydrolysis in one end of the molecule and a functional group such as carboxy group, amino group, epoxy group, aldehyde group, and the like in the other end, and binds with the inorganic material through an oxygen atom of the silanol group. Specific examples include mercaptopropyl triethoxysilane, glycidoxypropyl triethoxysilane, aminopropyl triethoxysilane, silane coupling agent including polyethylene glycol chain (for example, PEG-silane no. SIM6492.7 manufactured by Gelest Inc.), and the like. In a case in which the silane coupling agent is used, two or more kinds may be used together.

Well-known methods can be used as the reaction method between the fluorescent organic dye-containing silica particles and the silane coupling agent. For example, the obtained fluorescent organic dye-containing silica particles are dispersed in pure water, the aminopropyl triethoxysilane is added, and reaction is performed at room temperature for 12 hours. After the reaction ends, by centrifugal separation or filtration, it is possible to obtain fluorescent organic dye-containing silica particles having a surface modified with the aminopropyl group. Next, the amino group is reacted with the carboxy group in the antibody so that the antibody binds with the fluorescent organic dye-containing silica particles through amide bond. If necessary, condensing agent such as EDC (1-Ethyl-3-[3-Dimethylaminopropyl] carbodiimide Hydrochloride: manufactured by Pierce (Registered Trademark)) may also be used.

If necessary, a linker compound including a portion which can directly bind with the fluorescent organic dye-containing silica particles modified with the organic molecule and a portion which can bind with the molecular target substance can be used. For example, when sulfo-SMCC (Sulfosuccinimidyl 4[N-maleimidomethyl]-cyclohexane-1-carboxylate: manufactured by Pierce) which has a portion which selectively reacts with the amino group and a portion which selectively reacts with the mercapto group is used, the amino group of the fluorescent organic dye-containing silica particles modified with aminopropyl triethoxysilane and the mercapto group in the antibody are bound, and with this, the fluorescent organic dye-containing silica particles bound with the antibody is made.

When the biological material-recognizing portion is bound with the fluorescent substance-containing polystyrene particles, the same process as the quantum dot can be applied either the fluorescent substance is the fluorescent organic dye or the quantum dot. In other words, by impregnating the fluorescent organic dye and the quantum dot in the polystyrene particles with the functional group such as the amino group, it is possible to obtain the fluorescent substance-containing polystyrene particles with the functional group, and then by using the EDC or the sulfo-SMCC, the fluorescent substance-containing polystyrene particles bound with the antibody is made.

Examples of an antibody that recognizes a specific antigen include M. actin, M.S. actin, S.M. actin, ACTH, Alk-1, α1-antichymotrypsin, α1-antitrypsin, AFP, bcl-2, bcl-6, β-catenin, BCA 225, CA19-9, CA125, calcitonin, calretinin, CD1a, CD3, CD4, CD5, CD8, CD10, CD15, CD20, CD21, CD23, CD30, CD31, CD34, CD43, CD45, CD45R, CD56, CD57, CD61, CD68, CD79a, "CD99, MIC2", CD138, chromogranin, c-KIT, C-MET, collagen type IV, Cox-2, cyclin D1, keratin, cytokeratin (high molecular mass), pankeratin, pankeratin, cytokeratin 5/6, cytokeratin 7, cytokeratin 8, cytokeratin 8/18, cytokeratin 14, cytokeratin 19, cytokeratin 20, CMV, E-cadherin, EGFR, ER, EMA, EBV, VIII factor related antigen, fassin, FSH, galectin-3, gastrin, GFAP, glucagon, glycophorin A, granzyme B, hCG, hGH, helicobacterpyroli, HBc antigen, HBs antigen, hepatocyte specific antigen, HER2, HSV-I, HSV-II, HHV-8, IgA, IgG, IgM, IGF-1R, inhibin, insulin, kappa L chain, Ki67, lambda L chain, LH, lysozyme, macrophage, melan A, MLH-1, MSH-2, myeloperoxidase, myogenin, myoglobin, myosin, neurofilament, NSE, p27 (Kip1), p53, p53, p63, PAX 5, PLAP, pneumocystis calini, podoplanin (D2-40), PGR, prolactin, PSA, prostatic acid phosphatase, Renal Cell Carcinoma, S100, somatostatin, spectrin, synaptophysin, TAG-72, TdT, thyroglobulin, TSH, TTF-1, TRAcP, tryptase, villin, vimentin, WT1, Zap-70, and the like.

STAINING METHOD

The staining method for the tissue specimen will be described. The staining method described below can be applied not only to the tissue specimen but also to cells.

There is no limitation on methods of making a specimen to which the staining method described below is applied. A specimen made in known methods can be used.

i) REMOVING PARAFFIN

A tissue specimen is immersed in a container with xylene, and paraffin is removed. The temperature is not particularly limited, and the processing can be performed at room temperature. Preferably, the immersing time is 3 minutes or more and 30 minutes or less. The xylene can be changed during the immersion as necessary.

Next, the tissue specimen is immersed in a container with ethanol, and the xylene is removed. The temperature is not particularly limited, and the processing can be performed at room temperature. Preferably, the immersing time is 3 minutes or more to 30 minutes or less. The ethanol can be changed during the immersion as necessary.

Next, the tissue specimen is immersed in a container with water to remove the ethanol. The temperature is not particularly limited, and the processing can be performed at room temperature. Preferably, the immersing time is 3 minutes or more and 30 minutes or less. The water can be changed during the immersion as necessary.

ii) ACTIVATING PROCESSING

Activating processing of the target biological material in the tissue section is performed according to known methods. The activating conditions are not specifically set, and examples of liquid for activation that can be used include, 0.01 M citric acid buffered solution (pH 6.0), 1 mMEDTA solution (pH 8.0), 5% urea, 0.1 M tris-hydrochloric acid buffered solution. Examples of the heating device that can be used include autoclave, microwave, pressure pan, water bath, and the like. The temperature is not particularly limited, and the processing can be performed at room temperature. The processing can be performed at a temperature of 50 to 130 °C and the amount of time that the processing is performed can be 5 to 30 minutes.

Next, the tissue specimen after activating processing is immersed in the container with PBS (Phosphate Buffered Saline), and cleaning is performed. The temperature is not limited, and the processing can be performed at room temperature. Preferably, the immersing time is 3 minutes or more to 30 minutes or less. The PBS can be changed during the immersion as necessary.

iii) STAINING USING FLUORESCENT SUBSTANCE-ASSEMBLED PARTICLES BOUND WITH BIOLOGICAL MATERIAL-RECOGNIZING PORTION

The PBS dispersion liquid of the fluorescent substance-assembled particles bound with a biological material-recognizing portion is placed on the tissue specimen and reacted with the target biological material. By changing the biological material-recognizing portion bound with the fluorescent substance-assembled particles, staining can be applied to various biological materials. In a case in which the fluorescent substance-assembled particles bound with several kinds of biological material-recognizing portion are used, the fluorescent substance-assembled particles PBS dispersion liquid of each of the above may be mixed in advance, or the liquid may be sequentially placed on the tissue specimen separately.

The temperature is not particularly limited, and the processing can be performed at room temperature. Preferably, the reacting time is 30 minutes or more to 24 hours or less.

Preferably, a known blocking agent such as BSA included in PBS is dropped before staining with the fluorescent substance-assembled particles.

Next, the tissue specimen after staining is immersed in the container with PBS, and unreacted fluorescent substance-assembled particles are removed. The temperature is not particularly limited, and the processing can be performed at room temperature. Preferably, the immersing time is 3 minutes or more to 30 minutes or less. The PBS can be changed during the immersion as necessary. A cover glass is placed on the tissue specimen to be sealed. A commercially available sealing agent can be used as necessary.

H staining and DAB staining are performed before sealing with the cover glass.

OBTAINING FLUORESCENCE IMAGE

The microscopic image acquiring device 1A is used to obtain a wide field microscopic image (fluorescence image) of the stained tissue specimen. In the microscopic image acquiring device 1A, the excitation light source and the optical filter for fluorescence detection are suitably selected corresponding to the absorption maximum wavelength and the fluorescent wavelength of the fluorescent substance used in the fluorescent staining reagent.

A field of view of a fluorescence image is preferably 3mm$^2$ or more, more preferably 30mm$^2$ or more, and still more preferably 300mm$^2$ or more.

OPERATION OF PATHOLOGICAL DIAGNOSIS SUPPORT SYSTEM 100 (INCLUDING IMAGE PROCESSING METHOD)

Operations of obtaining the fluorescence image and the bright field image mentioned above and performing analysis in the pathological diagnosis support system 100 will be explained. In this example, a target of observation is the tissue specimen stained with the staining reagent that includes the fluorescent substance-assembled particles bound with the biological material-recognizing portion that recognizes a specific biological material (In this example, the biological material is Ki67 protein in breast cancer tissue; hereafter referred to as "specific protein"). However, the target of observation is not limited to this.

First, an operator stains the tissue specimen with three staining reagents, i.e. an H staining reagent, a DAB staining reagent, and a staining reagent which includes the fluorescent substance-assembled particles as a fluorescent labeling material. The fluorescent substance-assembled particles are bound with a biological material-recognizing portion that recognizes specific protein.

Subsequently, the microscopic image acquiring device 1A obtains the bright field image and the fluorescence image through the following steps (a1) to (a5).

(a1) The operator puts the tissue specimen stained with the H staining reagent, the DAB staining reagent, and the staining reagent including the fluorescent substance-assembled particles on a slide, and places the slide on a slide fixing stage of the microscopic image acquiring device 1A.
(a2) The bright field unit is set, magnification and focus are adjusted, and the observation target region in the tissue is positioned in a visual field.
(a3) The imaging unit performs imaging to generate an image data of the bright field image, and the image data is transmitted to the image processing device 2A.
(a4) The unit is changed to the fluorescent unit.
(a5) The imaging unit performs imaging without changing the visual field and the magnification to generate an image data of the fluorescence image, and the image data is transmitted to the image processing device 2A.

In the image processing device 2A, image analysis processing 1 is performed based on the bright field image and the fluorescence image.

FIG. 5 shows a flowchart of image analysis processing 1 in the image processing device 2A. The controller 21 performs image analysis processing 1 in FIG. 5 in coordination with programs stored in the memory 25.

First, the communicator I/F 24 inputs the bright field image from the microscopic image acquiring device 1A (Step S1: input step). The controller 21 extracts cell nucleus regions from the bright field image based on presence and absence of dye staining (Step S2).

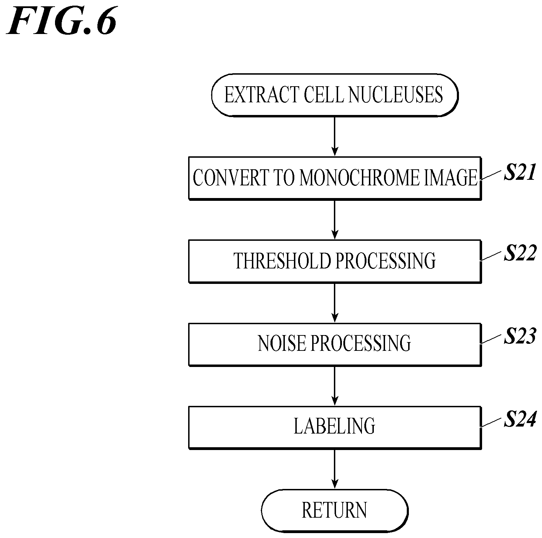

FIG. 6 shows a detailed flow of processing of Step S2. The controller 21 performs processing of Step S2 in coordination with programs stored in the memory 25.

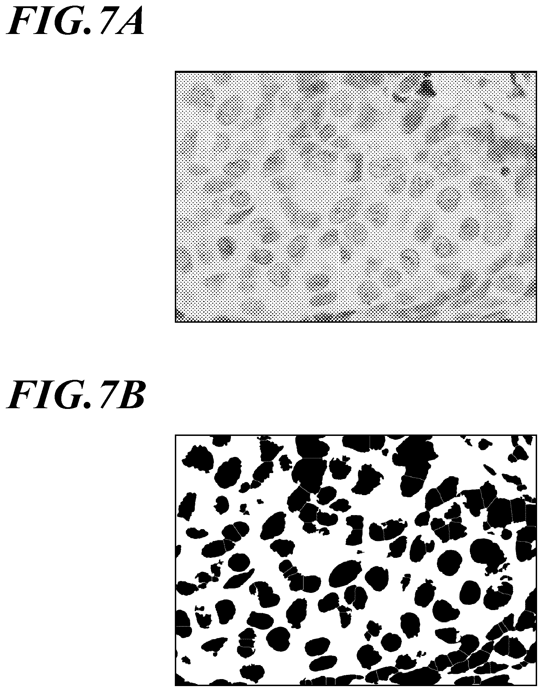

In Step S2, the controller 21 first converts the bright field image into a monochrome image (Step S21). FIG. 7A shows an example of the bright field image.

Then, threshold processing is performed on the monochrome image with a predetermined threshold to binarize pixel values (Step S22).

The controller 21 then performs noise processing (Step S23). Specifically, the noise processing can be performed by performing closing processing on the binary image. The closing processing is processing of performing dilation processing and then erosion processing by the same number of times. The dilation processing is processing of replacing the target pixel with a white pixel when any of the pixels within the range of n x n pixels (n is an integer of 2 or more) from the target pixel is white. The erosion processing is processing of replacing the target pixel with a black pixel when any of the pixels within the range of n x n pixels from the target pixel is black. Small regions such as noise can be removed by the closing processing. FIG. 7B shows an example of the image after the noise processing. As shown in FIG. 7B, an image (cell nucleus image) in which cell nucleuses are extracted is generated after the noise processing.

The controller 21 then performs labeling processing on the image after the noise processing. The extracted cell nucleuses are labeled respectively (Step S24). The labeling processing is processing of identifying objects in an image by giving the same labels (numbers) to connected pixels. Each cell nucleus in the image after the noise processing is identified and labeled through the labeling processing.

Subsequently, the controller 21 identifies cell types based on presence and absence of dye staining in the bright field image (Step S3). In the embodiment, immune cells are stained through DAB staining. For each cell nucleus extracted in Step S2, the controller 21 identifies presence and absence of DAB staining. Thus, stained immune cells and unstained cancer cells are distinguished.

On the other hand, the controller 21 generates a dye density image based on pixel values of pixels in the bright field image (Step S4: dye amount calculation).

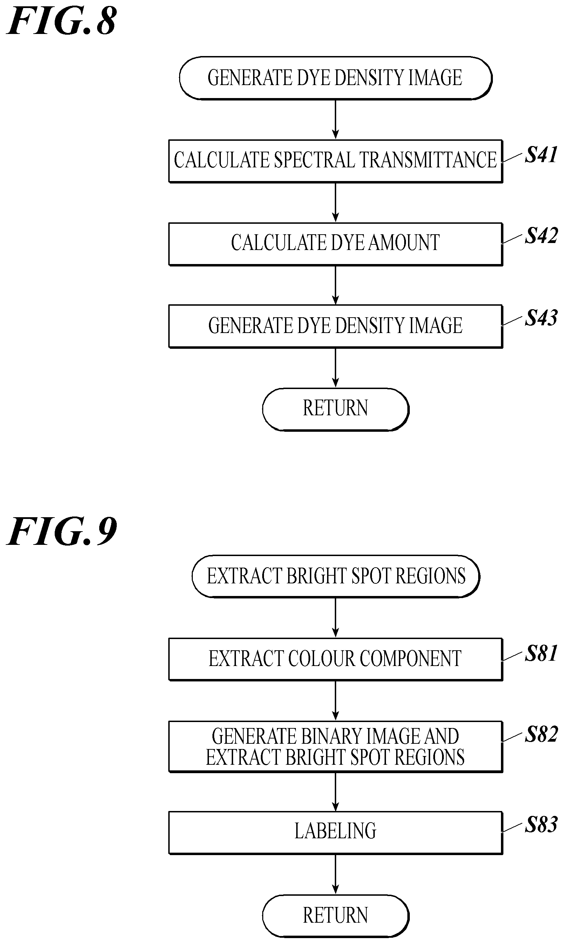

FIG. 8 shows a detailed flow of processing of Step S4. The controller 21 performs processing of Step S4 in coordination with programs stored in the memory 25.

First, the controller 21 calculates spectral transmittance of each wavelength at a point on the specimen corresponding to each pixel in the bright field image (Step S41). Techniques such as Wiener estimation are used.

Subsequently, the controller 21 calculates a dye amount at a point corresponding to a pixel on the tissue specimen for each pixel in the morphological cell image (Step S42). In the embodiment, H staining and DAB staining are performed on the tissue specimen. Therefore, the dye amount is calculated for each dye. Specifically, an equation between spectral transmittance and the dye amount according to Lambert-Beer's law is set up for each dye (in the embodiment, for each dye based on color development of the H staining reagent and the DAB staining reagent). The dye amounts are calculated by solving the equations simultaneously.

The controller 21 then generates a dye density image in which the dye amounts calculated for respective pixels are distributed to corresponding pixels in the bright field image (Step S43).

Thus, generation of the dye density image is finished.

On the other hand, after the communicator I/F 24 inputs the fluorescence image from the microscopic image acquiring device 1A (Step S5: input process), the controller 21 adds the dye density image generated in Step S4 to the fluorescence image (Step S6).

The controller 21 then adjusts fluorescence brightness in accordance with dye density (Step S7: brightness value adjustment).

This will be explained specifically. The dye of the staining reagent used to generate the bright field image absorb excitation light from the light source and fluorescence emitted from the fluorescent substance. Thereby brightness of the fluorescent substance attenuates in according with the dye amount. A fluorescence brightness value I is represented by the following equation (1), where "I" is brightness of the fluorescent substance-assembled particles that have attenuated through superposition with the dye, and "$I_0$" is brightness of the fluorescent substance-assembled particles before attenuation.

$$I = I_0 \exp(-Ad) \qquad (1)$$

The variable "d" is the dye amount calculated in Step S4. "A" is an attenuation coefficient and is a value that depends upon the fluorescent substance-assembled particles, a fluorescence wavelength, the dye of the staining reagent, imaging conditions, and the like.

Thus, the brightness value $I_0$ before attenuation is represented by the following equation (2).

$$I_0 = I/\exp(-Ad) \qquad (2)$$

The fluorescence brightness value $I_0$ calculated in the above equation (2) for each pixel in the fluorescence image is used for further analysis.

Subsequently, the controller 21 extracts bright spot regions from the fluorescence image (Step S8).

FIG. 9 shows a detailed flow of processing of Step S8. The controller 21 performs processing of Step S8 in coordination with programs stored in the memory 25.

In Step S8, first, the controller 21 extracts a color component corresponding to a wavelength of a fluorescent bright spot from the fluorescence image (Step S81).

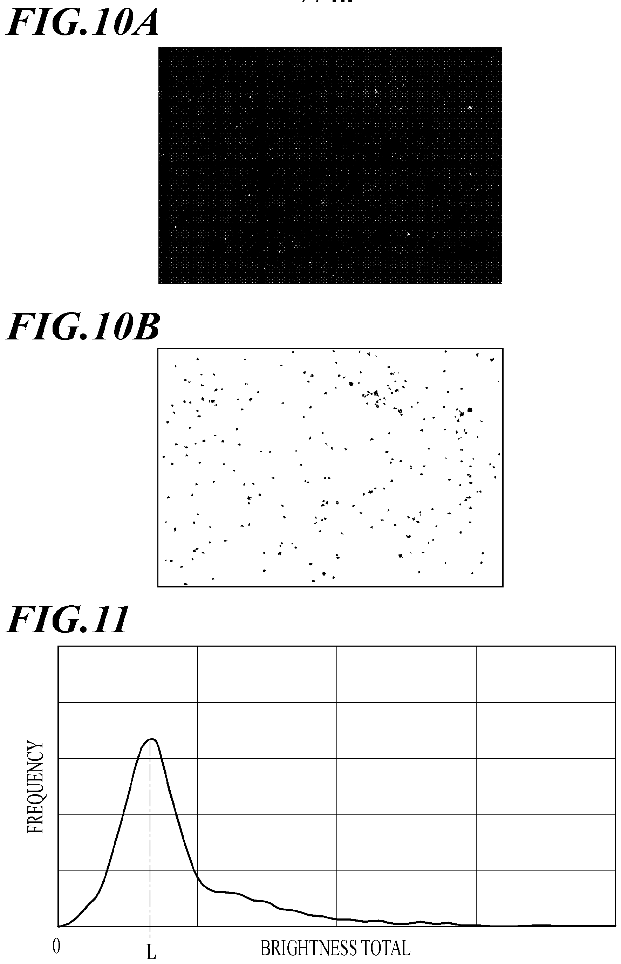

FIG. 10A shows an example of the fluorescence image.

In Step S81, in a case in which the emission wavelength of fluorescent particles is, for example, 550nm, only fluorescent bright spots having this wavelength component are extracted as an image.

The controller 21 then performs threshold processing on the extracted image to generate a binarized image and extracts bright spot regions (Step S82).

Processing of removing noises such as auto fluorescence of cells or other unwanted signal components may be performed before the threshold processing. Preferably, low-pass filters such as Gaussian filters and high-pass filters such as second differential are used.

FIG. 10B shows an example of an image in which the bright spot regions are extracted. As shown in FIG. 10B, bright spot regions mainly consisting of fluorescent bright spots are extracted in the image.

The controller 21 then performs labeling processing on the bright spot regions.
The extracted bright spot regions are labeled respectively (Step S83).

The controller 21 then calculates a brightness total of each bright spot region (Step S9: brightness value calculation). Specifically, after the image in which the bright spot regions are extracted is generated from the fluorescence image, the image in which the bright spot region are extracted is put on the fluorescence image of a portion corresponding to each bright spot region. The image in which the bright spot regions are extracted is used as a mask to generate the second fluorescence image corresponding to the bright spot regions from the fluorescence image. Brightness distribution in the X-coordinate and Y-coordinate is generated based on the second fluorescence image. The brightness total is the sum of these values.

Then, the controller 21 calculates the number of fluorescent particles in each bright spot region (Step S10: feature amount calculation). Specifically, first, a brightness distribution curve in FIG. 11 is generated based on the brightness total calculated in Step S9. The horizontal axis indicates brightness total. The vertical axis indicates frequency (ratio to the number of all the bright spots, or the number of bright spots). For example, the mode of the brightness total (brightness total L at the peak of the brightness distribution curve) is calculated as an average brightness value based on the brightness distribution curve. A brightness total in each bright spot region is divided by the average brightness value. The resulting value is the number of fluorescent dye-assembled particles, i.e. the number of fluorescent particles, in each bright spot region.

After processing of Step S3 and Step S10 are completed, the bright spot region image is added to the cell nucleus image (Step S11). In this step, it is effective to adjust positional deviation between the cell nucleus image and the bright spot region image.

Distribution of the bright spot regions on the cell nucleuses is then displayed on the display 23 of the image processing device 2A. Also, the number of fluorescent particles per cell nucleus is calculated for each cell type and is displayed on the display 23 (Step S12).

The calculation of the number of fluorescent particles for each cell type is to calculate the number of fluorescent particles respectively for immune cells and cancer cells identified in Step S3. Expression levels of specific proteins in the immune cells and the cancer cells are classified and are displayed on the display 23.

As described above, the pathological diagnosis support system 100 of the first embodiment performs image processing including:

inputting a fluorescence image and a morphological cell image that are obtained by imaging a tissue specimen in which:

a specific biological material is stained with a fluorescent substance capable of binding with the specific biological material; and
cells are stained with a dye capable of being observed under visible light;

extracting a predetermined region including a bright spot from the fluorescence image and calculating a brightness value of the predetermined region; and
adjusting the brightness value of the predetermined region based on dye information of the morphological cell image.

This suppresses influence of dyes in the tissue specimen to which fluorescent staining of a specific protein with the fluorescent substance and dye staining to visualize cells or a region of interest in cells are performed. The brightness value of the fluorescent substance is accurately detected. Therefore, an expression level of the specific protein is quantitatively assessed with higher accuracy.

In the pathological diagnosis support system 100 of the first embodiment, the dye amount is calculated without just using RGB values, chroma, etc. of the bright field image. It quantitatively suppresses influence of dye.

Such an adjustment method is particularly effective in a case in which the tissue specimen is subjected to multiple staining with several kinds of dyes having different wavelength characteristics. Since a pixel value is higher at a portion where dyes overlap with each other, if adjustment is performed using just pixel values, a brightness total becomes smaller than an actual value. However, according to the embodiment, the adjustment formula is set up for each dye so that the brightness value is adjusted more accurately.

In the pathological diagnosis support system 100 of the first embodiment, fluorescent staining is performed using the fluorescent substance-assembled particles in which fluorescent substances are assembled. Since the fluorescent substance-assembled particles have higher brightness and higher light resistance than a single fluorescent substance, presence of fluorescent substances is detected as bright spots. The fluorescent substance-assembled particles suit a case in which an expression level of a biological material is evaluated as in the present invention.

The number of fluorescent particles is calculated with high accuracy for each cell type. Thus, in the above example, the expression level of specific protein is identified for each of the immune cell and the cancer cell. This analysis technique provides a vital clue to show in detail how to treat patients.

MODIFICATION 1

In the above embodiment, the fluorescence brightness is adjusted in accordance with the dye amount. In Modification 1, the fluorescence brightness is adjusted in accordance with RGB pixel values.

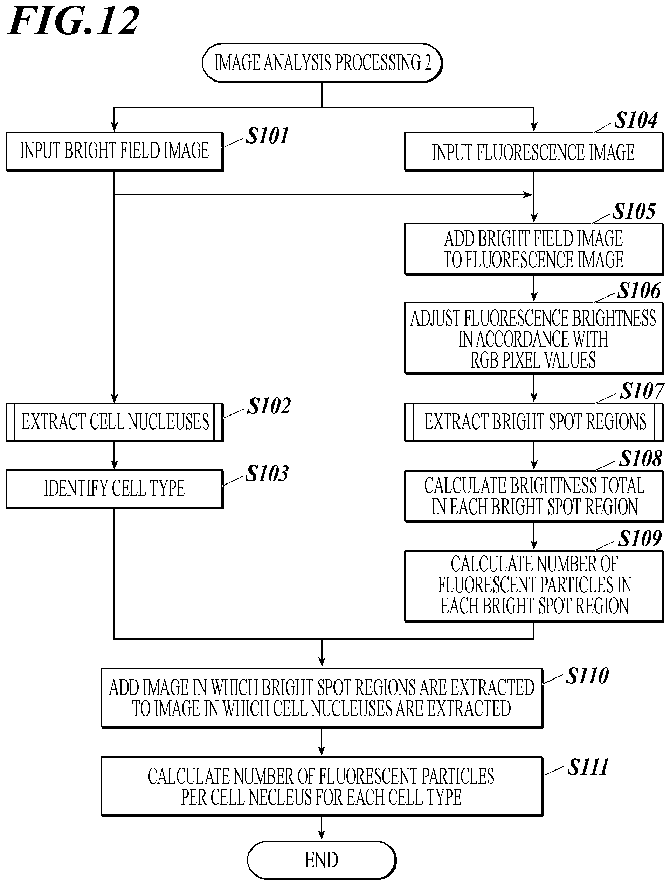

FIG. 12 shows a flow chart of image analysis processing 2 in the image processing device 2A of Modification 1. The controller 21 performs the image analysis processing 2 in FIG. 12 in coordination with programs stored in the memory 25.

Processing of Step S101 to Step S103 and Step S104 in FIG. 12 is similar to the processing of Step S1 to Step S3 and Step S5 in FIG. 5. Explanation is omitted.

In Step S105, the controller 21 adds the fluorescence image input in Step S104 to the bright field image input in Step S101.

The controller 21 then adjusts the fluorescence brightness in accordance with RGB pixel values (Step S106). The fluorescence brightness value $I_0$ before attenuation due to superposition of dye is represented by the following Equation (3), where "I" is the fluorescence brightness value after the attenuation, and "A" is the attenuation coefficient.

$$I_0 = I/\exp(-Ad_1) \qquad (3)$$

The variable $d_1$ is represented by the following equation (4), where "R", "G", and "B" are RGB pixel values of a pixel.

$$d_1 = aR+bG+cB \qquad (4)$$

The coefficients "a", "b", and "c" respectively correspond to "R", "G", and "B".

Subsequent analysis is performed using the fluorescence brightness value $I_0$ calculated in Equation (3) for each pixel in the fluorescence image.

Processing of Step S107 to Step S111 is similar to the processing of Step S8 to Step S12 in FIG. 5. Explanation is omitted.

MODIFICATION 2

In Modification 2, the fluorescence brightness is adjusted based on an HSV image.

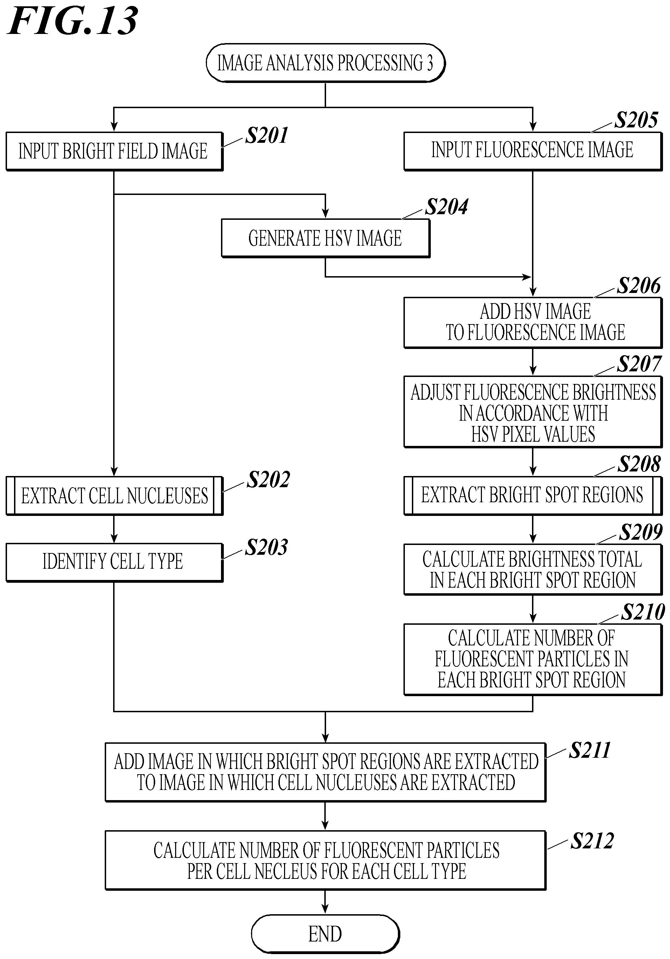

FIG. 13 shows a flow chart of image analysis processing 3 in the image processing device 2A of Modification 2. The controller 21 performs image analysis processing in FIG. 13 in coordination with programs stored in the memory 25.
Processing of Step S201 to Step S203 and Step S205 in FIG. 13 is similar to the processing of Step S1 to Step S3 and Step S5 in FIG. 5. Explanation is omitted.
In Step S204, the controller 21 converts RGB of the bright field image into HSV using a known formula to generate an HSV image (Step S204: chroma calculation).
The controller 21 then adds the fluorescence image input in Step S205 to the HSV image generated in Step S204 (Step S206).
The controller 21 then adjusts the fluorescence brightness in accordance with HSV pixel values (Step S207). The fluorescence brightness value $I_0$ before attenuation due to superposition of dye is represented by the following equation (5), where "I" is the fluorescence brightness value after the attenuation, and "A" is the attenuation coefficient.

$$I_0 = I/\exp(-Ad_2) \qquad (5)$$

The variable $d_2$ is represented by the following, where "S" is a chroma value of a pixel.

$$d_2 = S \qquad (6)$$

Thus, the fluorescence brightness value $I_0$ is adjusted based on chroma.
Processing of Step S208 to Step S212 is similar to the processing of Step S8 to Step S12 in FIG. 5. Explanation is omitted.
Similarly, adjustment using a Lab image or an LCH image may be applied as a method of adjusting the fluorescence brightness in accordance with chroma.
In a case in which the fluorescence brightness is adjusted based on a Lab image, the fluorescence brightness value $I_0$ before attenuation due to superposition of dye is represented by the following equation (7), where "I" is the fluorescence brightness value after the attenuation, and "A" is the attenuation coefficient.

$$I_0 = I/\exp(-Ad_3) \qquad (7)$$

The Variable $d_3$ is represented by the following equation (8).

$$d_3{\text -} = a^2 + b^2 \qquad (8)$$

Thus, $d_3$ represents chroma. The fluorescence brightness value $I_0$ is adjusted based on chroma.
Similarly, in a case in which the fluorescence brightness is adjusted based on an LCh image, the fluorescence brightness value $I_0$ before attenuation due to superposition of dye is represented by the following equation (9), where "I" is the fluorescence brightness value after the attenuation, and "A" is the attenuation coefficient.

$$I_0 = I/\exp(-Ad_4) \qquad (9)$$

The variable $d_4$ is represented by the following, where "C" is a chroma value of a pixel.

$$d_4 = C \qquad (10)$$

Thus, the fluorescence brightness value $I_0$ is adjusted based on chroma.
As shown in Modification 1 and Modification 2, the present invention can be applied to a wide range. The brightness value may be adjusted based on various color spaces.
It is a simple method to set up the adjustment formula directly from RGB values as in Modification 1 since the step of generating the dye density image or the step of converting the dye density image into the HSV image or the like can be omitted.

SECOND EMBODIMENT

Hereinafter, the second embodiment for carrying out the present invention will be described with reference to the drawings, but the present invention is not limited thereto. The same reference numerals are assigned to the same components as those of the first embodiment. Details are omitted.
In the first embodiment, the fluorescence brightness is adjusted before the bright spot regions are extracted from the fluorescence image. In the second embodiment, the number of fluorescent particles is adjusted based on the bright field image after the bright spot regions are extracted and the number of fluorescent particles is calculated.

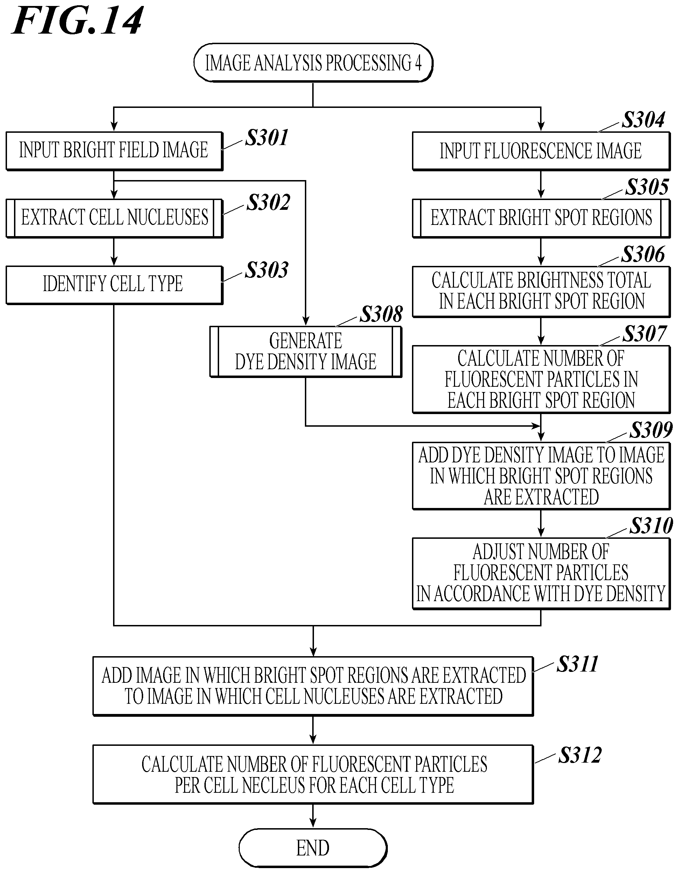

FIG. 14 shows a flowchart of image analysis processing 4 of the image processing device 2A according to the second embodiment. The controller 21 performs the image analysis processing 4 in FIG. 14 in coordination with programs stored in the memory 25.
Processing of Step S301 to Step S303, Step S304 and Step S305 to Step S307 in FIG. 14 is similar to the processing of Step S1 to Step S3, Step S5 and Step S8 to Step S10 in FIG. 5. Explanation is omitted.

In Step S308, the controller 21 generates the dye density image based on pixel values of pixels of the bright field image as in the first embodiment.

The controller 21 then adds the dye density image generated in Step S308 to the image which is generated in Step S307 and in which the bright spot regions are extracted (Step S309).

The controller 21 then adjusts the number of fluorescent particles in accordance with dye density (Step S310: feature amount adjustment)

Specifically, first, the fluorescence brightness value $I_0$ is calculated by adjusting the "I", which is the fluorescence brightness value after attenuation, based on the dye amount "d" as in the processing of Step S7 in the first embodiment. A ratio ($r = I_0/I$) of the fluorescence brightness value $I_0$ to the fluorescence brightness value I is calculated. The number of fluorescent particles calculated in Step S307 is divided by "r". Thus, the number of fluorescent particles is adjusted.

Processing of Step S311 and Step S312 is similar to the processing of Step S11 and Step S12 in FIG. 5. Explanation is omitted.

As described above, the pathological diagnosis support system 100 of the second embodiment performs image processing including:

inputting a fluorescence image and a morphological cell image that are obtained by imaging a tissue specimen in which:

a specific biological material is stained with a fluorescent substance capable of binding with the specific biological material; and
cells are stained with a dye capable of being observed under visible light;

extracting a predetermined region including a bright spot from the fluorescence image and calculating a brightness value of the predetermined region;
calculating a feature amount that indicates an expression level of the specific biological material based on the brightness value of the predetermined region; and
adjusting the feature amount based on dye information of the morphological cell image.

Accordingly, in a case in which brightness of a fluorescent bright spot is high enough to be detected, the same effect as the first embodiment is achieved by calculating the feature amount first and then adjusting the feature amount.

OTHER EMBODIMENTS

The description of the above embodiment is a preferable example of the present invention, but the present invention is not limited thereto.

For example, in the embodiment, the fluorescence brightness is adjusted by the adjustment formula. However, for example, a look-up table may be prepared to calculate an adjustment amount of the fluorescence brightness from RGB values of the bright field image

In the above embodiment, the fluorescent staining is performed with fluorescent substance-assembled particles of one kind, but the fluorescent substance-assembled particles may be other than this. The fluorescent staining may be performed with several kinds of fluorescent substances having different wavelength characteristics. In this case, the adjustment formula (the attenuation coefficient A) varies according to the fluorescent substance. Therefore, the adjustment formula should be set up for each combination of the fluorescent substance and the dye.

If the dye staining for the bright field image is performed before the fluorescent staining, the fluorescent substance-assembled particles are less likely to adhere to the biological material. Therefore, it is preferable to perform the dye staining after the fluorescent staining. In a case in which the dye staining is performed after the fluorescent staining, either adjustment method of the first embodiment and the second embodiment can be used. In a case in which the dye staining is performed before the fluorescent staining, it is preferable to adjust the number of fluorescent particles as in the second embodiment and to calculate the "r" by setting the adjustment formula in accordance with staining order.

In a case in which multiple dye staining is performed with dyes having different wavelength characteristics, it is preferable to set up the adjustment formula in accordance with staining order for adjustment.

In the above embodiment, the fluorescent substance-assembled particles are used as the fluorescent substance. The fluorescent substance-assembled particles have high brightness so that the number of bright spots is easily measured. However, the fluorescent substance is not limited to this. A single fluorescent dye or a single quantum dot may be used.

In the above embodiment, the cell nucleuses are extracted from the bright field image. Alternatively, any region of interest may be extracted.

Furthermore, the above description discloses an example which uses an HDD, a semiconductor nonvolatile memory, or the like as the computer readable medium of the program of the present invention, however, the present invention is not limited to the above. A portable recording medium such as a CD-ROM, and the like can be applied as other computer readable media. A carrier wave can be applied as the medium which provides the data of the program of the present invention through a communication line.

The image processing device does not necessarily consist of a single device.

It may consist of specialized devices for respective configurations, such as an input unit, a brightness calculator, and a brightness adjuster.

Other than the above, the detailed configuration and the detailed operation of each device composing the pathological diagnosis support system 100 can be suitably changed within the scope of the present invention.

INDUSTRIAL APPLICABILITY

The present invention may be applied to an image processing method, an image processing device, and a program.

REFERENCE SIGNS LIST

| 1A | Microscopic image acquiring device |
| 2a | Image processing device |
| 21 | Controller |
| 22 | Operator |
| 23 | Display |
| 24 | Communicator I/F |
| 25 | Memory |
| 26 | Bus |
| 3a | Cable |
| 100 | Pathological diagnosis support system |

**Claims**

1. An image processing method, comprising:

　　inputting a fluorescence image and a morphological cell image that are obtained by imaging a tissue specimen in which:

　　　　a specific biological material is stained with a fluorescent substance capable of binding with the specific biological material; and cells are stained with a dye capable of being observed under visible light;

　　extracting a predetermined region including a bright spot from the fluorescence image and calculating a brightness value of the predetermined region; and
　　adjusting the brightness value of the predetermined region based on dye information of the morphological cell image.

2. The image processing method according to claim 1, further comprising:

　　calculating a dye amount of a dye used to stain the morphological cell image for each pixel in the morphological cell image,
　　wherein, in adjusting the brightness value, the dye amount is used as the dye information.

3. The image processing method according to claim 2, wherein
in calculating the dye amount, the dye amount is estimated in a color deconvolution method based on a dye base of each pixel in the morphological cell image, and a dye density image in which the dye amount is distributed in relation to pixels of the morphological cell image is generated, and
in adjusting the brightness value, the brightness value is adjusted based on the dye density image.

4. The image processing method according to claim 1, further comprising:

　　calculating chroma of a dye used to stain the morphological cell image for each pixel in the morphological cell image,
　　wherein, in adjusting the brightness value, the brightness value is adjusted using the chroma as the dye information.

5. The image processing method according to claim 1, wherein, in adjusting the brightness value, RGB values are used as the dye information.

6. The image processing method according to any one of claims 1 to 5, wherein
the tissue specimen is stained with a fluorescent substance and a dye,
the fluorescent substance consists of a single kind of fluorescent substance or multiple kinds of fluorescent substances having different wavelength characteristics,
the dye consists of a single kind of dye or multiple kinds of dyes having different wavelength characteristics, and
in adjusting the brightness value, the brightness value is adjusted for each combination of the fluorescent substance and the dye based on the dye information of the dye.

7. The image processing method according to any one of claims 1 to 6, wherein
the tissue specimen is stained with multiple kinds of dyes having different wavelength characteristics, and
in adjusting the brightness value, the brightness value is adjusted differently depending on staining order of the multiple kinds of dyes.

8. An image processing method comprising:

　　inputting a fluorescence image and a morphological cell image that are obtained by imaging a tissue specimen in which:

a specific biological material is stained with a fluorescent substance capable of binding with the specific biological material; and cells are stained with a dye capable of being observed under visible light;

extracting a predetermined region including a bright spot from the fluorescence image and calculating a brightness value of the predetermined region;
calculating a feature amount that indicates an expression level of the specific biological material based on the brightness value of the predetermined region; and
adjusting the feature amount based on dye information of the morphological cell image.

9. The image processing method according to any one of claims 1 to 8, wherein the fluorescent substance consists of fluorescent substance-assembled particles in which a plurality of fluorescent substances are assembled.

10. An image processing device, comprising:

an input unit that inputs a fluorescence image and a morphological cell image that are obtained by imaging a tissue specimen in which:

a specific biological material is stained with a fluorescent substance capable of binding with the specific biological material; and cells are stained with a dye capable of being observed under visible light;

a brightness calculator that extracts a predetermined region including a bright spot from the fluorescence image and calculates a brightness value of the predetermined region; and
a brightness adjuster that adjusts the brightness value of the predetermined region based on dye information of the morphological cell image.

11. A program that makes a computer in an image processing device function as:

an input unit that inputs a fluorescence image and a morphological cell image that are obtained by imaging a tissue specimen in which:

a specific biological material is stained with a fluorescent substance capable of binding with the specific biological material; and cells are stained with a dye capable of being observed under visible light;

a brightness calculator that extracts a predetermined region including a bright spot from the fluorescence image and calculates a brightness value of the predetermined region; and
a brightness adjuster that adjusts the brightness value of the predetermined region based on dye information of the morphological cell image.

# FIG.1

100

| MICROSCOPIC IMAGE ACQUIRING DEVICE | 1A | 3A | IMAGE PROCESSING DEVICE | 2A |

# FIG.2

2A

CONTROLLER — 21

22 — OPERATION INTERFACE

24 — COMMUNICATOR I/F

23 — DISPLAY

25 — MEMORY

26

*FIG.3*

*FIG.4*

# FIG.5

```
                        ( IMAGE ANALYSIS PROCESSING 1 )

        INPUT BRIGHT FIELD IMAGE  ─S1              INPUT FLUORESCENCE IMAGE  ─S5

                              GENERATE DYE DENSITY IMAGE  ─S4

                                                    ADD DYE DENSITY IMAGE  ─S6
                                                    TO FLUORESCENCE IMAGE

                                                    ADJUST FLUORESCENCE BRIGHTNESS  ─S7
                                                    IN ACCORDANCE WITH DYE DENSITY

        EXTRACT CELL NUCLEUSES  ─S2                 EXTRACT BRIGHT SPOT REGIONS  ─S8

        IDENTIFY CELL TYPE  ─S3                     CALCULATE BRIGHTNESS TOTAL  ─S9
                                                    IN EACH BRIGHT SPOT REGION

                                                    CALCULATE NUMBER OF  ─S10
                                                    FLUORESCENT PARTICLES IN
                                                    EACH BRIGHT SPOT REGION

        ADD IMAGE IN WHICH BRIGHT SPOT REGIONS ARE EXTRACTED  ─S11
        TO IMAGE IN WHICH CELL NUCLEUSES ARE EXTRACTED

        CALCULATE NUMBER OF FLUORESCENT PARTICLES  ─S12
        PER CELL NECLEUS FOR EACH CELL TYPE

                              ( END )
```

# FIG.6

```
        ( EXTRACT CELL NUCLEUSES )
                    |
                    v
  ┌─────────────────────────────────┐
  │ CONVERT TO MONOCHROME IMAGE      │──S21
  └─────────────────────────────────┘
                    |
                    v
  ┌─────────────────────────────────┐
  │      THRESHOLD PROCESSING        │──S22
  └─────────────────────────────────┘
                    |
                    v
  ┌─────────────────────────────────┐
  │        NOISE PROCESSING          │──S23
  └─────────────────────────────────┘
                    |
                    v
  ┌─────────────────────────────────┐
  │           LABELING               │──S24
  └─────────────────────────────────┘
                    |
                    v
             (    RETURN    )
```

*FIG.7A*

*FIG.7B*

## FIG.8

```
GENERATE DYE DENSITY IMAGE
            │
            ▼
CALCULATE SPECTRAL TRANSMITTANCE ── S41
            │
            ▼
CALCULATE DYE AMOUNT ── S42
            │
            ▼
GENERATE DYE DENSITY IMAGE ── S43
            │
            ▼
         RETURN
```

## FIG.9

```
EXTRACT BRIGHT SPOT REGIONS
            │
            ▼
EXTRACT COLOUR COMPONENT ── S81
            │
            ▼
GENERATE BINARY IMAGE AND
EXTRACT BRIGHT SPOT REGIONS ── S82
            │
            ▼
         LABELING ── S83
            │
            ▼
         RETURN
```

## FIG.10A

## FIG.10B

## FIG.11

# FIG.12

```
        IMAGE ANALYSIS PROCESSING 2
```

INPUT BRIGHT FIELD IMAGE — S101

INPUT FLUORESCENCE IMAGE — S104

ADD BRIGHT FIELD IMAGE TO FLUORESCENCE IMAGE — S105

ADJUST FLUORESCENCE BRIGHTNESS IN ACCORDANCE WITH RGB PIXEL VALUES — S106

EXTRACT CELL NUCLEUSES — S102

EXTRACT BRIGHT SPOT REGIONS — S107

IDENTIFY CELL TYPE — S103

CALCULATE BRIGHTNESS TOTAL IN EACH BRIGHT SPOT REGION — S108

CALCULATE NUMBER OF FLUORESCENT PARTICLES IN EACH BRIGHT SPOT REGION — S109

ADD IMAGE IN WHICH BRIGHT SPOT REGIONS ARE EXTRACTED TO IMAGE IN WHICH CELL NUCLEUSES ARE EXTRACTED — S110

CALCULATE NUMBER OF FLUORESCENT PARTICLES PER CELL NECLEUS FOR EACH CELL TYPE — S111

END

# *FIG.13*

```
        ( IMAGE ANALYSIS PROCESSING 3 )
                        |
        +---------------+---------------+
        |                               |
   INPUT BRIGHT FIELD IMAGE  /S201   INPUT FLUORESCENCE IMAGE  /S205
        |                               |
        |                  GENERATE HSV IMAGE  /S204
        |                               |
        |                  ADD HSV IMAGE
        |                  TO FLUORESCENCE IMAGE  /S206
        |                               |
        |                  ADJUST FLUORESCENCE BRIGHTNESS
        |                  IN ACCORDANCE WITH
        |                  HSV PIXEL VALUES  /S207
        |                               |
   EXTRACT CELL NUCLEUSES  /S202   EXTRACT BRIGHT SPOT REGIONS  /S208
        |                               |
   IDENTIFY CELL TYPE  /S203    CALCULATE BRIGHTNESS TOTAL
        |                       IN EACH BRIGHT SPOT REGION  /S209
        |                               |
        |                  CALCULATE NUMBER OF
        |                  FLUORESCENT PARTICLES IN
        |                  EACH BRIGHT SPOT REGION  /S210
        |                               |
        +---------------+---------------+
                        |
   ADD IMAGE IN WHICH BRIGHT SPOT REGIONS ARE EXTRACTED
   TO IMAGE IN WHICH CELL NUCLEUSES ARE EXTRACTED  /S211
                        |
   CALCULATE NUMBER OF FLUORESCENT PARTICLES
   PER CELL NECLEUS FOR EACH CELL TYPE  /S212
                        |
                    ( END )
```

# FIG.14

```
        ┌─────────────────────────────┐
        │  IMAGE ANALYSIS PROCESSING 4 │
        └─────────────────────────────┘
```

INPUT BRIGHT FIELD IMAGE — *S301*

EXTRACT CELL NUCLEUSES — *S302*

IDENTIFY CELL TYPE — *S303*

GENERATE DYE DENSITY IMAGE — *S308*

INPUT FLUORESCENCE IMAGE — *S304*

EXTRACT BRIGHT SPOT REGIONS — *S305*

CALCULATE BRIGHTNESS TOTAL IN EACH BRIGHT SPOT REGION — *S306*

CALCULATE NUMBER OF FLUORESCENT PARTICLES IN EACH BRIGHT SPOT REGION — *S307*

ADD DYE DENSITY IMAGE TO IMAGE IN WHICH BRIGHT SPOT REGIONS ARE EXTRACTED — *S309*

ADJUST NUMBER OF FLUORESCENT PARTICLES IN ACCORDANCE WITH DYE DENSITY — *S310*

ADD IMAGE IN WHICH BRIGHT SPOT REGIONS ARE EXTRACTED TO IMAGE IN WHICH CELL NUCLEUSES ARE EXTRACTED — *S311*

CALCULATE NUMBER OF FLUORESCENT PARTICLES PER CELL NECLEUS FOR EACH CELL TYPE — *S312*

```
        ┌─────────┐
        │   END   │
        └─────────┘
```

## INTERNATIONAL SEARCH REPORT

| International application No. |
| --- |
| PCT/JP2019/007984 |

A.  CLASSIFICATION OF SUBJECT MATTER
Int.Cl.  G01N33/483(2006.01)i,  G01N21/64(2006.01)i,  G01N33/53(2006.01)i,
        G06T1/00(2006.01)i

According to International Patent Classification (IPC) or to both national classification and IPC

B.  FIELDS SEARCHED

Minimum documentation searched (classification system followed by classification symbols)
Int.Cl.  G01N33/483, G01N21/64, G01N33/53, G06T1/00

| Documentation searched other than minimum documentation to the extent that such documents are included in the fields searched | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580  (JDreamIII)
CAplus/MEDLINE/EMBASE/BIOSIS  (STN)

C.  DOCUMENTS CONSIDERED TO BE RELEVANT

| Category* | Citation of document, with indication, where appropriate, of the relevant passages | Relevant to claim No. |
| --- | --- | --- |
| A | WO 2017/110974 A1 (KONICA MINOLTA, INC.) 29 June 2017, paragraphs [0055]-[0065] & US 2019/0012786 A1, paragraphs [0131]-[0163] & EP 3396375 A1 | 1–11 |
| A | WO 2017/050788 A1 (MYCARTIS N. V.) 30 March 2017, abstract, claims & JP 2018-529947 A & US 2018/0275059 A1 & EP 3147650 A1 & CN 108139325 A | 1–11 |

☒  Further documents are listed in the continuation of Box C.　　☐  See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 23 May 2019 (23.05.2019) | 04 June 2019 (04.06.2019) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2019/007984 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | WO 2017/187717 A1 (NAGOYA UNIVERSITY) 02 November 2017, paragraphs [0092]-[0094] & EP 3450963 A1, paragraphs [0083]-[0088] & CN 109073556 A & US 2019/0137396 A1 | 1-11 |
| A | JP 2015-090471 A (SONY CORP.) 11 May 2015, paragraphs [0054]-[0069] & US 2016/0246045 A1, paragraphs [0069]-[0084] & WO 2015/068360 A1 & EP 3066513 A1 | 1-11 |
| A | WO 2012/035705 A1 (TOHOKU UNIVERSITY) 22 March 2012, abstract, claims & US 2013/0230866 A1, abstract, claims & EP 2618154 A1 & CN 103154741 A & JP 5682976 B2 | 1-11 |
| A | JP 2004-101354 A (OLYMPUS CORP.) 02 April 2004, paragraphs [0049]-[0066] & US 2005/0153356 A1, paragraphs [0049]-[0066] & WO 2004/023117 A1 & EP 1548423 A1 | 1-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012029342 A
- JP 2002514319 A
- US 4326008 A
- US 5326692 A

**Non-patent literature cited in the description**

- *Langmuir,* 1992, vol. 8, 2921
- *New Journal of Chemistry,* 2009, vol. 33, 561
- *Nature Biotechnology,* 2001, vol. 19, 631